# EUROPÄISCHE PATENTANMELDUNG

(11) **EP 4 571 774 A1**
(43) Veröffentlichungstag der Anmeldung: **18.06.2025**
(21) Anmeldenummer: 23216854.2
(22) Anmeldetag: 14.12.2023
(51) Int. Cl.: G16H 40/63

(54) **INFUSIONSPUMPENSYSTEM UND ZUGEHÖRIGES BETRIEBSVERFAHREN**

(71) Anmelder: B. Braun Melsungen AG, 34212 Melsungen (DE)
(72) Erfinder: Kever, Felix, 34125 Kassel (DE)
(74) Vertreter: Tergau & Walkenhorst Intellectual Property GmbH

(57) **Zusammenfassung**

Die Erfindung betrifft ein Infusionspumpensystem (2), das ohne aufwendige Nachrüstung oder Integration von Barcodescannern oder dergleichen einen zuverlässigen, fehlerarmen Workflow ermöglicht. Dies wird erreicht durch ein Infusionspumpensystem (2) mit
• einer Anzahl, insbesondere einer Mehrzahl von Infusionspumpen (4),
• einer zentralen Datenverarbeitungsvorrichtung (8),
• einer Anzahl, insbesondere einer Mehrzahl von Medikationscontainern (14), wobei
• die jeweilige Infusionspumpe (4) mit der Datenverarbeitungsvorrichtung (8) kommuniziert und einen Anschluss oder eine Aufnahme (28) für einen der Medikationscontainer (14) aufweist,
• die Datenverarbeitungsvorrichtung (8) eine Medikamentendatenbank (10) mit einer Mehrzahl von Medikamentendatensätzen (12) umfasst,
• dem jeweiligen Medikamentendatensatz (12) ein Kurzbestätigungscode (24) zugeordnet ist,
• der jeweilige Medikationscontainer (14) einem der Medikamentendatensätze (12) zugeordnet und mit dem entsprechenden Kurzbestätigungscode (24) beschriftet oder gekennzeichnet ist,
• der jeweiligen Infusionspumpe (4) ein Eingabeelement (30) für die Eingabe eines der Kurzbestätigungscodes (24) zugeordnet ist, und
• die jeweilige Infusionspumpe (24) eine Konfigurationseinheit (32) aufweist oder mit einer solchen kommuniziert, die anhand eines am Eingabeelement (30) eingegebenen Kurzbestätigungscodes (24) einen zugehörigen Medikamentendatensatz (12) aus der Medikamentendatenbank (10) oder einer lokalen Kopie davon abruft sowie darin konfigurierte medikamentenspezifische Einstellungen oder Pumpenfunktionen aktiviert.

## Beschreibung

Die Erfindung betrifft ein Infusionspumpensystem und ein zugehöriges Betriebsverfahren.

Die Medikamentendatenbank (Drug Library) von im klinischen Umfeld genutzten Infusionspumpen ermöglicht es, Medikations-spezifische Randbedingungen zu konfigurieren (für welchen Pumpentyp ist das Medikament zulässig? Grenzwerte für die Flussrate, vordefinierte Dosisraten je nach Patientenprofil, ...), erhöht die Sicherheit im laufenden Betrieb (Medikamentenname sichtbar im Pumpendisplay, ggf. mit passendem Colorlabel) und ist Voraussetzung für eine entsprechende Dokumentation der Infusionstherapie im System der elektronischen Patientenakte (Electronic Medical Record oder kurz EMR).

Bei der manuellen Programmierung der Pumpe muss die korrekte Medikation über das User Interface der Pumpe aus der auf der Pumpe vorhandenen Medikamentendatenbank ausgewählt werden. Auch wenn hierbei Filter- und Auswahlfunktionen oder vorkonfigurierte Presets zum Einsatz kommen können, ist dieser Auswahlprozess mit einem bestimmten Aufwand verbunden und in einem gewissen Maß auch fehleranfällig.

Eine Verbesserung - allerdings verbunden mit zusätzlichem Hardware- und damit Kostenaufwand - bietet die lokale Einbindung eines Barcode-Scanners, der in diesem Fall meist direkt von der Pumpe gesteuert wird oder direkt mit der Pumpe interagiert. Ein Barcode-Scanner ist typischerweise ein Handgerät zur Erfassung von Strichcodes (1D) oder Matrix-Codes (2D) oder ein SmartDevice mit Kamera und entsprechender Bildauswertung. In Kombination mit entsprechenden, maschinenlesbaren Labels auf den Medikations-Containern, typischerweise Spritzen oder Infusionsbeutel, kann der Scanner den Medikationsnamen (und ggf. die Variante) einfach erfassen und einen automatischen Lookup in der Medikamentendatenbank der Pumpe auslösen, so dass der manuelle Auswahlprozess entfällt.

Damit sind unter anderem folgende Nachteile verbunden:
- Ein mit dem jeweiligen Racksystem verbundener Barcodescanner oder ein entsprechendes SmartDevice müssen an jedem Bettplatz verfügbar und betriebsbereit sein. Bei dem Racksystem handelt es sich um ein Stecksystem für mehrere Pumpen; dieses kann zu turmartigen Strukturen zusammengesetzt werden und bietet u. U. weitere Kommunikationsschnittstellen. Alternativ muss ein Scanner oder SmartDevice von der Pflegekraft mitgeführt werden und ist entweder jedes Mal physisch mit dem entsprechenden Racksystem zu verbinden, oder drahtlos zu koppeln oder muss (z. B. bei Verbindung mit einem Backend) temporär dem Racksystem zugeordnet werden.
- Während es bei Racksystemen oftmals Anschlussmöglichkeiten für derartige Scanner gibt, ist die Verbindung mit einzeln genutzten und drahtlos mit dem EMR-System vernetzten Infusionspumpen in den seltensten Fällen möglich oder praktikabel.

Der Erfindung liegt die Aufgabe zugrunde, ein Infusionspumpensystem anzugeben, das auch ohne aufwendige Nachrüstung oder Integration von Barcodescannern oder dergleichen einen zuverlässigen, fehlerarmen Workflow mit automatisiertem Abruf der Medikamentation in einer Medikamentendatenbank und entsprechender Programmierung der Infusionspumpe ermöglicht. Des Weiteren soll ein entsprechendes Verfahren zum Betreiben eines Infusionspumpensystems angegeben werden.

Die genannte Aufgabe wird erfindungsgemäß gelöst durch ein Infusionspumpensystem mit den Merkmalen des Anspruchs 1 und durch ein zugehöriges Verfahren gemäß Anspruch 10.

Demnach ist ein Infusionspumpensystem vorgesehen mit
- einer Anzahl, insbesondere einer Mehrzahl von Infusionspumpen,
- einer zentralen, übergeordneten Datenverarbeitungsvorrichtung (Backend),
- einer Anzahl, insbesondere einer Mehrzahl von Medikationscontainern,
   wobei
- die jeweilige Infusionspumpe mit der Datenverarbeitungsvorrichtung kommuniziert und einen Anschluss oder eine Aufnahme für einen der Medikationscontainer aufweist,
- die Datenverarbeitungsvorrichtung eine Medikamentendatenbank mit einer Mehrzahl von Medikamentendatensätzen umfasst,
- dem jeweiligen Medikamentendatensatz ein Kurzbestätigungscode zugeordnet ist,
- der jeweilige Medikationscontainer einem der Medikamentendatensätze zugeordnet und mit dem entsprechenden Kurzbestätigungscode beschriftet oder gekennzeichnet ist, sprich eine derartige Beschriftung aufweist,
- der jeweiligen Infusionspumpe ein Eingabeelement für die Eingabe eines der Kurzbestätigungscodes zugeordnet ist, und
- die jeweilige Infusionspumpe eine Konfigurationseinheit aufweist oder mit einer solchen kommuniziert, die anhand eines am Eingabeelement eingegebenen Kurzbestätigungscodes einen zugehörigen Medikamentendatensatz aus der Medikamentendatenbank oder einer lokalen Kopie davon abruft sowie darin konfigurierte, medikamentenspezifische Einstellungen oder Pumpenfunktionen aktiviert.

In bevorzugter Ausgestaltung ist der Kurzbestätigungscode ein alphanumerischer Code, insbesondere ein zwei-, drei oder vierstelliger Code, wobei jede der Stellen ein lateinischer Großbuchstabe von A bis Z oder eine der Ziffern 0 bis 9 sein kann. Zur Vermeidung von Verwechslungen können der Buchstabe "O" und ggf. auch "I" aus der zur Verfügung stehenden Grundmenge von Zeichen ausgelassen werden. Alternativ oder zusätzlich ist die Verwendung von Kleinbuchstaben möglich. Solch ein Code ist von einem menschlichen Benutzer gut lesbar und im Kurzzeitgedächtnis zu behalten, bietet gleichwohl eine relativ große Zahl einzigartiger bzw. eindeutiger Kombinationen.

Weiterhin ist es vorteilhaft, wenn die Beschriftung einen Aufdruck auf dem Medikationscontainer oder auf einem dort aufgebrachten Aufkleber oder an einem daran angebrachten Label umfasst. Es aber auch andere Ausgestaltungen der Beschriftung oder Kennzeichnung denkbar, etwa durch Prägung, Ausstanzung etc.

Vorteilhafterweise ist in der Datenverarbeitungsvorrichtung implementierten Medikamentendatenbank-Manager ein zusätzlicher Kurzcode-Manager implementiert, der die Kurzbestätigungscodes generiert und den Medikamentendatensätzen zuordnet. Durch die automatisierte Generierung werden Doubletten und Fehlzuordnungen vermieden.

In weiterer zweckmäßiger Ausgestaltung umfasst die die jeweilige Infusionspumpe eine Programmbibliothek mit einer Mehrzahl von Infusionsprorammen, die einzeln oder in geeigneter Kombination auswählbar sind, insbesondere in automatisierter Weise durch die integrierte Konfigurationseinheit. In einer denkbaren Alternative kann die Infusionspumpe das benötigte Programm von einer Programmbibliothek der Datenverarbeitungsvorrichtung herunterladen.

In einer bevorzugten Weiterentwicklung ist das Eingabeelement für die Eingabe eines weiteren Datenelements, welches in einer Beschriftung des jeweiligen Medikationscontainers enthalten ist, ausgelegt, wobei die Konfigurationseinheit eine Verifikationsroutine aufweist, die anhand des weiteren Datenelements die korrekte Eingabe des auf dem Medikationscontainer angegebenen Kurzbestätigungscodes überprüft. Dadurch wird auf einfache Weise eine Zwei-Faktor Verifikation implementiert, die Fehlzuordnungen noch zuverlässiger vermeidet.

Schließlich ist das Eingabeelement vorteilhafterweise in die Infusionspumpe integriert, insbesondere als Bildschirmtastatur in einer graphischen Benutzeroberfläche implementiert. Alternativ kann eine physische Tastatur verwendet werden. Denkbar ist auch die Eingabe über eine Tastatur oder Bildschirmtastatur eines zugeordneten und gekoppelten SmartDevice (Mobiltelefon, Tablet etc.).

Das in Bezug auf die Vorrichtung Gesagte lässt sich sinngemäß auf das Verfahren übertragen und umgekehrt.

Zusammenfassend löst das hier beschriebene Systemkonzept / der Workflow des "Code-based Medication Lookup" (CML) das eingangs genannte Problem mit Hilfe eines alphanumerischen Kurbestätigungscodes (Short Verification Code oder kurz SVC), welcher vom menschlichen Nutzer einfach und ohne technische Hilfsmittel zu erfassen und beispielsweise über ein dediziertes Soft-Keyboard im Rahmen der Programmierung an der Pumpe einzugeben ist.

Bereits ein zweistelliger alphanumerischer Code bietet rund 1.200 Ausprägungen, was für die überwiegende Zahl der Medikamentendatenbanken ausreichen dürfte. Für den unwahrscheinlichen Fall, dass ein zweistelliger SVC nicht ausreicht, können dreistellige Codes verwendet werden, was angesichts von in diesem Fall rund 42.000 Codevarianten einer beliebigen Skalierbarkeit gleichkommt.

Dieser medikationsspezifische, eindeutige Code sollte bevorzugt in die Medikamentendatenbank aufgenommen werden und zudem auf den Labels der Medikations-Container enthalten sein.

Zur Vermeidung von Fehlern oder Doubletten sollte das Erzeugen und Anlegen der Codevarianten eine Funktion in einem Medikamentendatenbank-Manager (Drug Library Manager oder kurz DLM) sein, was typischerweise eine Software-Komponente oder ein computerimplementierter Algorithmus ist, der in der Datenverarbeitungsvorrichtung abläuft.

Optional lässt sich über einen Zwei-Faktor-Lookup zusätzliche Sicherheit erzeugen, indem versehentliche Fehleingaben mit hoher Wahrscheinlichkeit identifiziert und somit effektiv ausgeschlossen werden. Hierzu fordert in einer bevorzugten Implementierung die Infusionspumpe den Benutzer nach der Eingabe des SVC und vor der Anzeige des Lookup-Ergebnisses auf, zusätzlich ein weiteres, vom Label des Medikations-Containers abzulesendes Datenelement einzugeben (etwa den Anfangsbuchstaben des Medikationsnamens, eine zusätzliche Kontrollnummer oder auch den jeweiligen Folgebuchstaben bzw. die Folgeziffer etc.). Fehleingaben des SVC können auf diese Weise sicher erkannt werden, ohne dass die Usability nennenswert leidet (zumal die Nutzung dieses Mechanismus kritischen Medikamenten vorbehalten sein kann).

Zwar ist festzuhalten, dass der zusätzliche Aufdruck des Medikamentennamens in Klarschrift auf dem Medikations-Label diesen Check ohnehin anbietet, durch den Zwei-Faktor-Lookup wird er jedoch verbindlich in den Workflow integriert und auch dokumentiert.

Zu den allgemeinen Voraussetzungen des CML-Konzepts gehört:
- Das Infusionspumpensystem enthält eine Medikamentendatenbank, die auf den Infusionspumpen zugänglich ist.
- Es sollte sichergestellt sein, dass diese Datenbank (insbesondere die Instanzen auf den Infusionspumpen) aktuell ist und inhaltlich mit der Datenbank übereinstimmt, die zum Drucken der Labels für die Medikationscontainer genutzt wird.

Die CML-bedingten Neuerungen und Anforderungen im Infusionspumpensystem lassen wie folgt charakterisieren:
- Das Datenmodell der Medikamentendatenbank innerhalb des Infusionspumpensystems muss um den SVC ergänzt werden.
- Sofern die Medikationsdatenbank sowohl in der Datenverarbeitungsvorrichtung als auch lokal auf den Pumpen verfügbar ist, müssen die SVCs der einzelnen Medikamente in sämtlichen Instanzen verfügbar sein.
- Es sollte bevorzugt eine Software-Komponente geben, welche eindeutige SVCs generiert und den Medikamenten in einer 1:1 Beziehung zuordnet. Es ist vorteilhaft, diese Komponente in einem zentralen, in der Datenverarbeitungsvorrichtung angeordneten Medikamentendatenbank-Manager zu integrieren.
- Die Infusionspumpe muss in der Lage sein, über die Eingabe eines SVC die dazugehörige Medikation aus der lokal oder in der Datenverarbeitungsvorrichtung verfügbaren Medikamentendatenbank herauszusuchen.
- Für einen optionalen "Zwei-Faktor-Lookup" muss die Pumpe zusätzlich in der Lage sein, einen weiteren Datenpunkt des jeweiligen Medikations-Datensatzes abzufragen (z. B. Anfangsbuchstabe des Namens, Medikamentenkategorie, etc.) und zum Abgleich des Suchergebnisses heranzuziehen.

Vorteile des CML-Systemkonzeptes sind unter anderem:
- Für die Programmierung von Infusionspumpen unter Nutzung der Medikamentendatenbank ist weder ein manuelles Heraussuchen von Datensätzen aus der Datenbank noch ein Scan-Prozess erforderlich, der wiederum entsprechende Hardware sowie weitere Arbeitsschritte benötigen würde. Stattdessen muss die Pflegekraft in der bevorzugten Version lediglich einen zwei- oder dreistelligen alphanumerischen Code an der Pumpe eingeben.
- Durch den Wegfall der zusätzlichen Hardware ist eine Implementierung wesentlich einfacher und schneller umzusetzen. Auch der Wartungsaufwand und die Zahl der möglichen Fehlerquellen reduzieren sich.
- Optional kann der Datenbank-Lookup durch Nutzung einer Zwei-Faktor-Authentifizierung zusätzlich abgesichert werden.
- Aus Sicht eines Krankenhauses oder sonstigen Behandlungszentrums lässt sich die Anzahl und Schwere von Medikationsfehlern in der Infusionstherapie durch konsequente Nutzung der Medikamentendatenbank deutlich reduzieren. Das CML-Konzept ermöglicht diese Nutzung nahezu ohne Mehraufwand an medizinischer Hardware oder IT-Infrastruktur.

Ein Ausführungsbeispiel der Erfindung wird nachfolgend unter Bezugnahme auf die beigefügte Zeichnung näher erläutert.

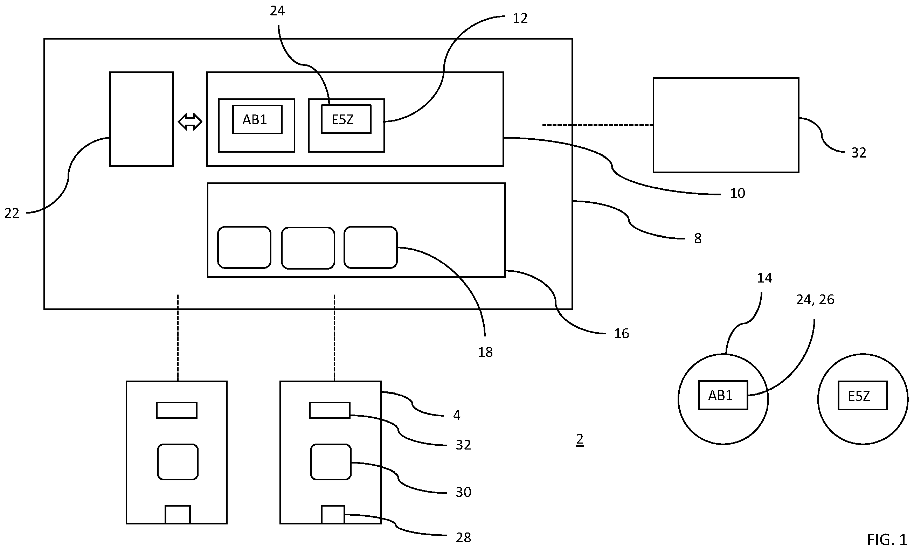

Darin zeigt die einzige Figur, nämlich FIG. 1, eine schematische Darstellung eine Infusionspumpensystems.

Das in FIG. 1 schematisch dargestellte Infusionspumpensystem 2 umfasst eine Mehrzahl von medizinischen Infusionspumpen 4, die beispielsweis kabelgebunden und/oder drahtlos mit einer elektronischen Datenverarbeitungsvorrichtung 8 kommunizieren. Die Datenverarbeitungsvorrichtung 8 enthält eine Medikamentendatenbank 10 (Drug Library) mit einer Mehrzahl von Medikamentendatensätzen 12, die zu Medikamenten korrespondieren, die in Medikationscontainern 14, etwa in Gestalt von Spritzen oder Infusionsbeuteln, bereitgestellt werden. Die jeweilige Infusionspumpe 4 enthält eine Programmbibliothek mit Infusionsprogrammen oder Routinen für eine Programmierung der Infusionspumpen 4. Des Weiteren kann die Datenverarbeitungsvorrichtung 8 eine Parameterbibliothek 16 mit Randbedingungen oder Parametern 18 enthalten, die bei der Programmierung oder Konfiguration der jeweiligen Infusionspumpe 4 angewendet werden. Diese Parameter 18 können Bestandteile der Medikamentendatensätze 12 sein, und die Parameterbibliothek 16 kann Bestandteil der Medikamentendatenbank 10 sein. Beispielsweise kann es sich bei einem Parameter 18 um einen medikamentenspezifischen Infusionsraten-Grenzwert oder eine erlaubte Bonusrate von Infusionslösung während einer Infusion handeln (d. h. beispielsweise eine manuell aktivierbare Zugabe zu einer geplanten Menge). Das heißt, es besteht eine vorzugsweise in der Datenverarbeitungsvorrichtung 8 hinterlegte Korrespondenz zwischen Medikamentendatensätzen 12 und infusionsbezogenen Parametern 18.

Die Medikationscontainer 14 werden vom medizinischen Personal zum jeweils vorgesehenen Infusionsplatz gebracht und dort an die Infusionspumpe 4 angeschlossen. Nachdem der Infusionspumpe 4 das zum Einsatz kommende Medikament mittgeteilt wurde und das passende Programm auf der Infusionspumpe 4 aktiviert wurde, erfolgt die Infusion am Patienten. Zur Vermeidung von Fehlzuordnungen (falsches Medikament, falsches Programm) ist eine Reihe von Maßnahmen vorgesehen, die im Folgenden näher beschrieben werden.

Die Datenverarbeitungsvorrichtung 8 umfasst dazu einen softwaremäßig implementierten Medikamentendatenbank-Manager 22 (Drug Library Manager oder kurz DLM), der zu jedem Medikamentendatensatz 12 in bevorzugt ein-eindeutiger Weise einen Kurzbestätigungscode 24 (Short Verification Code oder kurz SVC) im Sinne einer eindeutigen Identifizierung generiert und zuordnet (Unique Identifier). Dabei handelt es sich bevorzugt um einen zwei-, drei- oder maximal vierstelligen alphanumerischen Code. Bereits mit zwei alphanumerischen Zeichen (zweckmäßigerweise umfassend lateinische Großbuchstaben A bis Z ohne O sowie die Ziffern von 0 bis 9) bietet solch ein Code bzw. eine Kodierung rund unterschiedliche 1.200 Ausprägungen, bei drei Zeichen sind es rund 42.000. In FIG. 1 sind die zwei Fälle AB1 und E5Z als Beispiele dargestellt. Der Kurzbestätigungscode 24 ist bevorzugt Bestandteil des jeweiligen Medikamentendatensatzes 12.

Die den Medikamentendatensätzen 12 zugeordneten Medikationscontainer 14 sind entsprechend in menschenlesbarer Weise mit den Kurzbestätigungscodes 24 beschriftet, etwa durch unmittelbaren Aufdruck, Prägung, Gravierung oder dergleichen, oder durch Befestigung oder Anbringung entsprechend von beschrifteten Aufklebern, Etiketten, Labels oder dergleichen. Darüber hinaus kann die Beschriftung 26 als weiteren Beschriftungsbestandteil beispielsweise den Medikamentennamen im Klartext, eine zusätzliche Kontrollnummer oder dergleichen enthalten. Die Beschriftung 26 kann in automatisierter Weise durch ein Beschriftungssystem 34, etwa ein Druckwerk, erfolgen, welches auf den aktuellen Datenbestand der Medikamentendatenbank 10 zugreift.

Nachdem ein Mitglied des medizinischen Personals den jeweiligen Medikamentencontainer 14 zum vorgesehenen Infusionsplatz transportiert und mit dem Anschluss oder der Aufnahme 28 der zugeordneten Infusionspumpe 4 verbunden hat, aktiviert er/sie die Programmierung der Infusionspumpe 4. Dazu liest er/sie den Kurzbestätigungscode 24 und gegebenenfalls ein weiteres Datenelement, das sich von der weiteren Beschriftung 26 ableitet (beispielweise der Anfangsbuchstabe des Medikamentennamens oder die Kontrollnummer) vom Medikationscontainer 14 ab und gibt sie über ein der Infusionspumpe 4 zugeordnetes Eingabeelement 30 ein. Das Eingabeelement 30 ist bevorzugt eine in die Infusionspumpe 4 integriertes Tastatur oder ein Tastenfeld, insbesondere eine Bildschirmtastatur (Soft-Keyboard) auf einer zu Bedienung der Infusionspumpe 4 vorgesehenen graphischen Benutzerschnittstelle (Graphical User Interface oder kurz GUI).

Die Infusionspumpe 4 ruft nun mittels einer eingebauten oder zugeordneten Konfigurationseinheit 32 anhand des Kurzbestätigungscodes 24 den zugehörigen Medikamentendatensatz 12 aus der Medikamentendatenbank 10 ab (Lookup) und lädt gegebenenfalls zugehörige Parameter 18 aus der Parameterbibliothek 16 der Datenverarbeitungsvorrichtung 8 herunter, die auf die in der Infusionspumpe 4 hinterlegten Infusionsprogramme angewendet werden. Dann kann der Infusionsvorgang gestartet werden oder startet automatisch.

Falls ein weiteres Datenfeld im Sinne eines Zwei-Faktor-Lookups oder einer Checksumme verwendet wird, ist dem Abruf des Medikamentendatensatzes 12 eine Prüfung bzw. Verifikation des Kurzbestätigungscodes 24 anhand des weiteren Datenfeldes vorgeschaltet, wobei bei Nichtübereinstimmung eine Fehlermeldung ausgegeben und der weitere Ablauf zumindest temporär blockiert wird.

Die jeweilige Infusionspumpe 4 weist standardmäßig bevorzugt eine lokale Kopie der Medikamentendatenbank 10 auf, die beispielsweise durch periodische oder ereignisgesteuerte Synchronisation mit einer Master-Datenbank in der Datenverarbeitungsvorrichtung 8 aktuell gehalten wird. Der Abruf des Medikamentendatensatzes 12 auf der Basis des eingegebenen Kurzbetätigungscodes 24 (ggf. mit vorheriger Zwei-Faktor Verifikation) kann dann durch eine geeignete Routine lokal innerhalb der Infusionspumpe 4 erfolgen.

Zusammenfassend wird durch den Code- bzw. Kodierungs-basierten Abruf oder das Nachschlagen (Lookup) der Medikation in der Medikationsdatenbank 10 ein fehlerarmer und effizienter Workflow geschaffen, der auch bei Bestandssystemen ohne umfangreiche Hardwareerweiterungen, insbesondere ohne Integration zusätzlicher Scanner oder Barcodeleser, unter Nutzung von in der Regel ohnehin vorhandenen Komponenten wie einer graphischen Benutzerschnittstelle implementiert werden kann. Die notwendigen Erweiterungen sind vielmehr primär softwaremäßiger Natur und damit vergleichsweise einfach realisierbar.

### Bezugszeichenliste

- 2: Infusionspumpensystem
- 4: Infusionspumpe
- 8: Datenverarbeitungsvorrichtung
- 10: Medikamentendatenbank
- 12: Medikamentendatensatz
- 14: Medikationscontainer
- 16: Parameterbibliothek
- 18: Parameter
- 22: Medikamentendatenbank-Manager
- 24: Kurzbestätigungscode
- 26: Beschriftung
- 28: Aufnahme
- 30: Eingabeelement
- 32: Konfigurationseinheit
- 34: Beschriftungssystem

## Patentansprüche

1. Infusionspumpensystem (2) mit
• einer Anzahl, insbesondere einer Mehrzahl von Infusionspumpen (4),
• einer zentralen Datenverarbeitungsvorrichtung (8),
• einer Anzahl, insbesondere einer Mehrzahl von Medikationscontainern (14),
wobei
• die jeweilige Infusionspumpe (4) mit der Datenverarbeitungsvorrichtung (8) kommuniziert und einen Anschluss oder eine Aufnahme (28) für einen der Medikationscontainer (14) aufweist,
• die Datenverarbeitungsvorrichtung (8) eine Medikamentendatenbank (10) mit einer Mehrzahl von Medikamentendatensätzen (12) umfasst,
• dem jeweiligen Medikamentendatensatz (12) ein Kurzbestätigungscode (24) zugeordnet ist,
• der jeweilige Medikationscontainer (14) einem der Medikamentendatensätze (12) zugeordnet und mit dem entsprechenden Kurzbestätigungscode (24) beschriftet oder gekennzeichnet ist,
• der jeweiligen Infusionspumpe (4) ein Eingabeelement (30) für die Eingabe eines der Kurzbestätigungscodes (24) zugeordnet ist, und
• die jeweilige Infusionspumpe (24) eine Konfigurationseinheit (32) aufweist oder mit einer solchen kommuniziert, die anhand eines am Eingabeelement (30) eingegebenen Kurzbestätigungscodes (24) einen zugehörigen Medikamentendatensatz (12) aus der Medikamentendatenbank (10) oder einer lokalen Kopie davon abruft sowie darin konfigurierte medikamentenspezifische Einstellungen oder Pumpenfunktionen aktiviert.

2. Infusionspumpensystem (2) nach Anspruch 1, wobei der Kurzbestätigungscode (24) ein alphanumerischer Code ist.

3. Infusionspumpensystem (2) nach Anspruch 2, wobei der Kurzbestätigungscode (24) zwei-, drei oder vierstellig ist.

4. Infusionspumpensystem (2) nach einem der vorangehenden Ansprüche, wobei die Beschriftung (26) oder Kennzeichnung mit dem Kurzbestätigungscode (24) einen Aufdruck auf dem Medikationscontainer (14) oder auf einem dort aufgebrachten Aufkleber oder an einem daran angebrachten Label umfasst.

5. Infusionspumpensystem (2) nach einem der vorangehenden Ansprüche, wobei in der der Datenverarbeitungsvorrichtung (8) ein Medikamentendatenbank-Manager (22) implementiert ist, der die Kurzbestätigungscodes (24) generiert und den Medikamentendatensätzen (12) zuordnet.

6. Infusionspumpensystem (2) nach einem der vorangehenden Ansprüche, wobei die jeweilige Infusionspumpe (4) eine Programmbibliothek mit einer Mehrzahl von auswählbaren Infusionsprogrammen umfasst.

7. Infusionspumpensystem (2) nach einem der vorangehenden Ansprüche, wobei das Eingabeelement (30) für die Eingabe eines weiteren Datenelements, welches in einer Beschriftung (26) des jeweiligen Medikationscontainers (14) enthalten ist, ausgelegt ist, und wobei die Konfigurationseinheit (32) eine Verifikationsroutine aufweist, die anhand des weiteren Datenelements die korrekte Eingabe des Kurzbestätigungscodes (24) überprüft.

8. Infusionspumpensystem (2) nach einem der vorangehenden Ansprüche, wobei das Eingabeelement (30) in die Infusionspumpe (24) integriert ist.

9. Infusionspumpensystem (2) nach einem der vorangehenden Ansprüche, wobei das Eingabeelement (30) als Bildschirmtastatur in einer graphischen Benutzeroberfläche implementiert ist.

10. Verfahren zum Betreiben eines Infusionspumpensystems (2) mit
• einer Anzahl, insbesondere einer Mehrzahl von Infusionspumpen (4),
• einer zentralen Datenverarbeitungsvorrichtung (8),
• einer Anzahl, insbesondere einer Mehrzahl von Medikationscontainern (14), wobei
• die jeweilige Infusionspumpe (4) mit der Datenverarbeitungsvorrichtung (8) kommuniziert und einen Anschluss oder eine Aufnahme (28) für einen der Medikationscontainer (14) aufweist,
• die Datenverarbeitungsvorrichtung (8) eine Medikamentendatenbank (10) mit einer Mehrzahl von Medikamentendatensätzen (12) umfasst,
• der jeweiligen Infusionspumpe (4) ein Eingabeelement (30) für die Eingabe eines Kurzbestätigungscodes (24) zugeordnet ist,
mit den folgenden Schritten:
• dem jeweiligen Medikamentendatensatz (12) wird ein Kurzbestätigungscode (24) zugeordnet,
• der jeweilige Medikationscontainer (14) wird einem der Medikamentendatensätze (12) zugeordnet und mit dem entsprechenden Kurzbestätigungscode (24) beschriftet,
• die jeweilige Infusionspumpe (4) ruft anhand eines am Eingabeelement (30) eingegebenen Kurzbestätigungscodes (24) einen zugehörigen Medikamentendatensatz (12) aus der Medikamentendatenbank (10) oder einer lokalen Kopie davon ab und aktiviert darin konfigurierte medikamentenspezifische Einstellungen oder Pumpenfunktionen.
